# EUROPÄISCHE PATENTANMELDUNG

(11) **EP 3 075 706 A1**
(43) Veröffentlichungstag der Anmeldung: **05.10.2016**
(21) Anmeldenummer: 16000582.3
(22) Anmeldetag: 10.03.2016
(51) Int. Cl.: C01B 3/38

(54) **VERFAHREN UND EINE ANLAGE ZUR ERZEUGUNG VON SYNTHESEGAS**

(30) Priorität: 31.03.2015 DE 102015004213
(71) Anmelder: Linde Aktiengesellschaft, 80331 München (DE)
(72) Erfinder: Ranke, Harald, 82343 Pöcking (DE); Seliger, Andreas, 80796 München (DE); Göke, Volker, 82538 Geretsried (DE); Kandziora, Christine, 82056 Baierbrunn (DE); Mabrouk, Rachid, 81477 München (DE)
(74) Vertreter: Fischer, Werner

(57) **Zusammenfassung**

Die Erfindung betrifft ein Verfahren zur Erzeugung von Synthesegas (21, 22), das insbesondere zu Herstellung von kohlenwasserstoffhaltigen Treibstoff, Ammoniak oder Harnstoff bereitgestellt wird. Das Verfahren umfasst die Schritte: Bereitstellen eines ersten Einsatzgasstromes (11,12) umfassend Methan und Umsetzen des ersten Einsatzgasstromes (11,12) mit Wasserdampf (29) in einem Reformierungsschritt (31) unter Erhalt eines Synthesegastroms (21, 22) umfassend CO und H₂. Erfindungsgemäß ist vorgesehen, dass aus dem Einsatzgasstrom (11) vor dem Reformierungsschritt (31) mindestens ein erster Teilstrom (13) abgetrennt wird, der erste Teilstrom (13) anschließend mit einem zweiten Einsatzgasstrom (15) umfassend mindestens 95 Vol. % Sauerstoff zu einem Abgasstrom (17) umfassend CO₂ und Wasser verbrannt wird, und der Abgasstrom (17) zumindest zu einem Teil in den Einsatzgasstrom (12) nach Abtrennen des ersten Teilstroms (13) zurückgeführt wird. Die Erfindung betrifft weiterhin eine Anlage zur Erzeugung von Synthesegas (21, 22).

## Beschreibung

Die Erfindung betrifft ein Verfahren und eine Anlage zur Erzeugung von Synthesegas.

Ein solches Verfahren umfasst die Schritte: Bereitstellen eines ersten Einsatzgasstromes umfassend Methan und Umsetzen des ersten Einsatzgasstromes mit Wasserdampf in einem Reformierungsschritt unter Erhalt eines Synthesegasstroms umfassend CO und H₂.

Die Dampf-Methan-Reformierungs-(steam *methane reforming*-SMR)-Technologie ist eine bekannte und etablierte Technologie, die seit Jahrzehnten zur Herstellung von Synthesegas verwendet wird. Vor allem ist die SMR-Technologie gegenüber anderen Verfahren besonders zur Wasserstoffgewinnung geeignet, wobei der ohnehin schon hohe Wasserstoffgehalt des durch SMR erhältlichen Synthesegases zusätzlich durch eine Wassergas-Shift-Reaktion gesteigert werden kann.

Zur Herstellung von Synthesegas per se haben sich jedoch auch andere Techniken wie etwa die partielle Oxidation (POX) oder die autothermale Reformierung (ATR) als mindestens konkurrenzfähig, wenn nicht sogar geeigneter als die SMR-Technologie erwiesen, insbesondere bei Herstellung großer Mengen, bei der sich unerwünschte Nebenprodukte der SMR-Technologie als nachteilig erweisen können. Auf der anderen Seite erfordern POX- oder ATR-Verfahren Sauerstoff, wobei der Sauerstoffbedarf mit dem Wasserstoff-Gehalt des Synthesegases steigt.

Basierend auf diesem Hintergrund ist es die der Erfindung zugrunde liegende Aufgabe, ein einfaches und wirtschaftliches Verfahren zur Herstellung von Synthesegas zur Verfügung zu stellen, das insbesondere durch eine hohe Kohlenstoffeffizienz und reduzierte Kohlendioxidemission gekennzeichnet ist.

Diese Aufgabe wird dadurch gelöst, dass aus dem Einsatzgasstrom vor dem Reformierungsschritt ein erster Teilstrom abgetrennt wird, der anschließend mit einem zweiten Einsatzgasstrom umfassend mindestens 95 Vol. % Sauerstoff zu einem Abgasstrom umfassend CO₂ und Wasser umgesetzt wird, und der Abgasstrom zumindest zu einem Teil in den Einsatzgasstrom nach dem Abtrennen des ersten Teilstromes zurückgeführt wird.

Vorteilhafterweise wird der Reformierungsschritt in einem Dampfreformer durchgeführt, wobei der Dampfreformer mindestens ein Reformerrohr und eine Brennkammer umfasst und dazu ausgebildet ist, die in der Brennkammer entstehende Wärme auf das zumindest eine Reformerrohr zu übertragen. Das Reformerrohr ist mit einem geeigneten Katalysator ausgestaltet, wie zum Beispiel einem Nickelkatalysator.

Aus der Rückführung des zumindest einen Teils des Abgases ergeben sich mehrere Vorteile: die Ausbeute an Synthesegas wird erhöht, die CO₂ Emission wird reduziert und die Kohlenstoffeffizienz des Verfahrens wird erhöht. Des Weiteren ist kein Import von externem CO₂ notwendig, um ggf. das H₂/CO-Verhältnis zu verschieben.

Bei dem Abgasstrom handelt es sich insbesondere um einen CO₂-reichen Gasstrom, der einen CO₂-Gehalt von mindestens 50 Vol. % aufweist, insbesondere einen CO₂-Gehalt von 54 Vol. % bis 75 Vol. %, bevorzugt einen CO₂-Gehalt von 61 Vol. %. Ein solcher Gasstrom kann vorteilhaft in Verfahren eingesetzt werden, die CO₂ benötigen, wie beispielsweise zur Synthese von Harnstoff oder zur *enhanced oil recovery*, wobei CO₂ unter hohem Druck in ein Bohrloch geführt wird, um das Öl an die Oberfläche zu drücken.

Gemäß einer Ausführungsform der Erfindung ist vorgesehen, dass der Einsatzgasstrom vor dem Reformierungsschritt und vor dem Abtrennen des ersten Teilstromes entschwefelt wird, wobei schwefelhaltige Verbindungen aus dem Einsatzgasstrom entfernt werden.

Gemäß einer Ausführungsform der Erfindung ist vorgesehen, dass der Reformierungsschritt bei einer Temperatur von 750°C bis 950°C und bei einem Druck von 10 bar bis 40 bar, vorzugsweise bei einem Druck von 30 bar durchgeführt wird.

Gemäß einer Ausführungsform der Erfindung ist vorgesehen, dass die bei der Verbrennung des ersten Teilstroms entstehende Wärme auf den Reformierungsschritt übertragen wird. Vorteilhafterweise wird dadurch Wärme für den endothermen Reformierungsschritt bereitgestellt.

Gemäß einer weiteren Ausführungsform der Erfindung ist vorgesehen, dass der Abgasstrom zu einem anderen Teil in den ersten Teilstrom zurückgeführt wird. Vorteilhafterweise kann dadurch einerseits die Flammentemperatur kontrolliert werden. Anderseits führt die Anreicherung der Brennkammer mit CO₂ dazu, dass die Wärme durch die nun vermehrte IR-Strahlung der CO₂ Moleküle effizienter auf den Reformationsschritt bzw. auf das Reformerrohr übertragen wird.

Gemäß einer weiteren Ausführungsform der Erfindung ist vorgesehen, dass der zweite Einsatzgasstrom umfassend mindestens 95 Vol. % Sauerstoff durch die Gastrennung von Luft bereitgestellt wird. Insbesondere wird durch die vorgenannte Gastrennung ein dritter Einsatzgasstrom zur Verfügung gestellt, der im Wesentlichen Stickstoff umfasst. Ein im Wesentlichen Stickstoff umfassender Einsatzgasstrom ist im Sinne der vorliegenden Beschreibung ein Gasstrom, der Stickstoffgehalt von mindestens 98 Vol. %, 99 Vol. %, 99,9 Vol. % oder 99,99 Vol. % aufweist.

Gemäß einer weiteren Ausführungsform der Erfindung ist vorgesehen, dass der Synthesegasstrom umfassend CO und H₂ unter Erzeugung von Wasserdampf auf eine Temperatur von 50°C bis 70°C gekühlt wird. Vorteilhafterweise wird durch diese Ausführungsform der für den Reformierungsschritt notwendige Wasserdampf zur Verfügung gestellt. Der erzeugte Wasserdampf kann vorteilhafterweise jedoch auch in anderen Verfahren eingesetzt werden, wie etwa zur Stromerzeugung. Gemäß einer weiteren Ausführungsform der Erfindung ist vorgesehen, dass der Synthesegasstrom umfassend CO und H₂ anschließend wieder auf eine Temperatur von 150°C bis 220°C erhitzt wird.

Gemäß einer weiteren Ausführungsform der Erfindung ist vorgesehen, dass aus dem Synthesegasstrom umfassend CO und H₂ zumindest ein zweiter Teilstrom abgetrennt wird, der in einem Wassergas-Shift-Reaktionsschritt zu einem Rohwasserstoffstrom umgesetzt wird, wobei CO und Wasser zu CO₂ und Wasserstoff umgesetzt wird. Vorteilhafterweise wird dadurch eine größere Menge an elementarem Wasserstoff bereitgestellt, der für eine Vielzahl von Verfahren wie etwa der Ammoniaksynthese verwendet werden kann.

Gemäß einer weiteren Ausführungsform der Erfindung ist vorgesehen, dass aus dem Rohwasserstoffstrom unter Erhalt eines ersten Produktstroms, der im Wesentlichen H₂ umfasst, ein erster Restgasstrom, der im Wesentlichen CO₂, und optional H₂, nicht umgesetztes CO und/oder nicht umgesetztes Methan umfasst, abgetrennt wird, und der erste Restgasstrom in den ersten Teilstrom zurückgeführt wird. Ein im Wesentlichen H₂ umfassender Produktstrom ist im Sinne der Erfindung durch einen Wasserstoffgehalt von mindestens 99 Vol. %, 99,9 Vol. % oder 99,99 Vol. % gekennzeichnet.

Gemäß einer weiteren Ausführungsform der Erfindung ist vorgesehen, dass der erste Restgasstrom durch Druck-Wechsel-Absorption unter Verwendung eines im Wesentlichen H₂-haltigen Spülgases abgetrennt wird, wobei der Restgasstrom zusätzlich H₂ umfasst. Vorteilhafterweise wird das H₂-haltige Spülgas zumindest zu einem Teil durch das erste Produktgas bereitgestellt. Ein im Wesentlich H₂ umfassendes Spülgas bezeichnet im Sinne der Erfindung einen Gasstrom, der einen Wasserstoffgehalt von mindestens 98 Vol. %, 99 Vol. %, 99,9 Vol. % oder 99,99 Vol. % aufweist. Vorteilhaferweise kann der im Restgasstrom enthaltene Wasserstoff in die Brennkammer des Reformers zurückgeführt werden, um dort durch Verbrennung die für den Reformierungsschritt erforderliche Wärme zu erzeugen.

Gemäß einer weiteren Ausführungsform der Erfindung ist vorgesehen, dass der Synthesegasstrom umfassend CO und H₂ in einem Fischer-Tropsch-Syntheseschritt zu einem Rohproduktstrom umfassend eine Mischung aus mindestens einem leichten C₁-C₄ Kohlenwasserstoff, mindestens einem schweren Kohlenwasserstoff mit mehr als 4 Kohlenstoffatomen und nicht umgesetztem Synthesegas umfassend CO und H₂ umgesetzt wird.

Gemäß einer weiteren Ausführungsform der Erfindung ist vorgesehen, dass dem Rohproduktstrom ein zweiter Restgasstrom umfassend den mindestens einen leichten C₁-C₄ Kohlenwasserstoff und das nicht umgesetzte Synthesegas abgetrennt wird, und der zweite Restgasstrom in den Einsatzgasstrom zurückgeführt wird. Vorteilhafterweise wird der zweite Restgasstrom ohne weitere Aufarbeitung direkt in den Einsatzgasstrom geführt. Durch diese Verdünnung des Einsatzgasstromes durch den zweiten Restgasstrom wird der CO-Partialdruck im Einsatzgas reduziert, was die Haltbarkeit des Katalysators im Reformerrohr deutlich erhöht.

Gemäß einer weiteren Ausführungsform der Erfindung ist vorgesehen, dass aus dem zweiten Restgasstrom ein zweiter Teilstrom abgetrennt wird, und der zweite Teilstrom in den Fischer-Tropsch-Syntheseschritt zurückgeführt wird. Vorteilhafterweise kann dadurch die Ausbeute des Fischer-Tropsch-Syntheseschrittes erhöht werden, da das nicht umgesetzte Synthesegas erneut der Reaktion zugeführt wird.

Gemäß einer weiteren Ausführungsform der Erfindung ist vorgesehen, dass aus dem zweiten Restgasstrom ein dritter Teilstrom abgetrennt wird, der unter Erzeugung von Wasserdampf verbrannt wird, wobei das resultierende Abgas der Verbrennung zumindest zum Teil in den Reformierungsschritt bzw. den Dampfreformer zurückgeführt wird, insbesondere über den zweiten Restgasstrom.

Gemäß einer weiteren Ausführungsform der Erfindung ist vorgesehen, dass der erste, im Wesentlichen H₂ umfassende Produktstrom zumindest zum Teil in den Rohproduktstrom geführt wird, insbesondere in den Rohproduktstrom nach Abtrennen des zweiten Restgasstromes. Vorteilhafterweise können dadurch sauerstoffhaltige oder ungesättigte Kohlenwasserstoffe zu gesättigten Kohlenwasserstoffen reduziert werden.

Gemäß einer alternativen Ausführungsform der Erfindung ist vorgesehen, dass der erste, im Wesentlichen H₂ umfassende Produktstrom zur Synthese von Ammoniak bereitgestellt wird, wobei der synthetisierte Ammoniak insbesondere zur Synthese von Harnstoff bereitgestellt wird.

Gemäß einer weiteren alternativen Ausführungsform der Erfindung ist vorgesehen, dass der dritte Einsatzgasstrom umfassend im Wesentlichen Stickstoff zur Synthese von Ammoniak bereitgestellt wird.

Gemäß einer weiteren alternativen Ausführungsform ist vorgesehen, Ammoniak mit CO₂ zu Harnstoff umzusetzen. Vorteilhafterweise wird CO₂ dabei von einem Teil des Abgasstromes zur Verfügung gestellt.

Gemäß einem weiteren Aspekt der Erfindung wird ein Verfahren zur Herstellung von Ammoniak zu Verfügung gestellt. Ein solches Verfahren umfasst die Schritte:
- Bereitstellen eines ersten Einsatzgasstromes umfassend Methan,
- Umsetzen des ersten Einsatzgasstromes mit Wasserdampf in einem Reformierungsschritt unter Erhalt eines Synthesegasstroms umfassend CO und H₂, wobei
   - aus dem ersten Einsatzgasstrom vor dem Reformierungsschritt mindestens ein erster Teilstrom abgetrennt wird,
   - der erste Teilstrom anschließend mit einem zweiten Einsatzgasstrom umfassend mindestens 95 Vol. % Sauerstoff zu einem Abgasstrom umfassend CO₂ und Wasser verbrannt wird,
   - der Abgasstrom zumindest zu einem Teil in den ersten Einsatzgasstrom stromauf des Reformierungsschritts zurückgeführt wird,
- Umsetzen von zumindest einem Teil des Synthesegasstroms umfassend CO und H₂ in einer Wassergas-Shift-Reaktion zu einem Rohwasserstoffstrom, wobei CO und Wasser zu CO₂ und H₂ umgesetzt werden, und optional Abtrennen eines ersten Produktstromes umfassend im Wesentlichen H₂ aus dem Rohwasserstoffstrom,
- Umsetzen von Wasserstoff und Stickstoff zu Ammoniak, wobei der Wasserstoff durch den Rohwasserstoffstrom oder ersten Produktstrom umfassend im Wesentlichen H₂ bereitgestellt wird.

Gemäß einer weiteren Ausführungsform der Erfindung ist vorgesehen, dass der zweite Einsatzgasstrom durch die Gastrennung von Luft bereitgestellt wird, wobei durch die Gastrennung zusätzlich ein dritter, im Wesentlichen Stickstoff umfassender Einsatzgasstrom bereitgestellt wird, und der oben bezeichnete, für die Ammoniakherstellung vorgesehene Stickstoff durch den dritten Einsatzgasstrom zur Verfügung gestellt wird.

Gemäß einer weiteren Ausführungsform der Erfindung ist vorgesehen, dass aus dem Rohwasserstoffstrom neben dem ersten Produktstroms ein erster Restgasstrom abgetrennt wird, der im Wesentlichen CO₂, und optional H₂, nicht umgesetztes CO und/oder nicht umgesetztes Methan umfasst, und der erste Restgasstrom in den ersten Teilstrom zurückgeführt wird.

Gemäß einer weiteren Ausführungsform der Erfindung ist vorgesehen, dass der erste Restgasstrom durch Druck-Wechsel-Absorption unter Verwendung eines H₂-haltigen Spülgases abgetrennt wird, so dass der Restgasstrom zusätzlich H₂ umfasst.

Vorteilhaferweise kann der im Restgasstrom enthaltene Wasserstoff in die Brennkammer des Reformers zurückgeführt werden, um dort durch Verbrennung die für den Reformierungsschritt erforderliche Wärme zu erzeugen.

Gemäß einem weiteren Aspekt der Erfindung wird ein Verfahren zur Herstellung von Harnstoff zu Verfügung gestellt. Das Verfahren umfasst die Schritte:
- Bereitstellen eines ersten Einsatzgasstromes umfassend Methan,
- Umsetzen des ersten Einsatzgasstromes mit Wasserdampf in einem Reformierungsschritt unter Erhalt eines Synthesegasstroms umfassend CO und H₂, wobei
   - aus dem ersten Einsatzgasstrom vor dem Reformierungsschritt mindestens ein erster Teilstrom abgetrennt wird,
   - der erste Teilstrom anschließend mit einem zweiten Einsatzgasstrom umfassend mindestens 95 Vol. % Sauerstoff zu einem Abgasstrom umfassend CO₂ und Wasser verbrannt wird,
   - der Abgasstrom zumindest zu einem Teil in den ersten Einsatzgasstrom stromauf des Reformierungsschritts zurückgeführt wird,
- Umsetzen von zumindest einem Teil des Synthesegasstroms umfassend CO und H₂ in einer Wassergas-Shift-Reaktion zu einem Rohwasserstoffstrom, wobei CO und Wasser zu CO₂ und H₂ umgesetzt werden, und optional Abtrennen eines ersten Produktstromes umfassend im Wesentlichen H₂ aus dem Rohwasserstoffstrom,
- Umsetzen von Wasserstoff und Stickstoff zu Ammoniak, wobei der Wasserstoff durch den Rohwasserstoffstrom oder ersten Produktstrom umfassend im Wesentlichen H₂ bereitgestellt wird,
- Umsetzen des Ammoniaks und CO₂ zu Harnstoff.

Dabei entsteht Harnstoff gemäß folgenden Reaktionen:

NH₃ + CO₂ ←→ H₂NCOONH₄

H₂NCOONH₄ ←→ (NH₂)₂CO + H₂O

Gemäß einer weiteren Ausführungsform der Erfindung ist vorgesehen, dass der zweite Einsatzgasstrom durch die Gastrennung von Luft bereitgestellt wird, wobei durch die Gastrennung zusätzlich ein dritter, im Wesentlichen Stickstoff umfassender Einsatzgasstrom bereitgestellt wird, und der oben bezeichnete für die Ammoniakherstellung vorgesehene Stickstoff durch den dritten Einsatzgasstrom zur Verfügung gestellt wird.

Gemäß einer Ausführungsform der Erfindung wird das CO₂ durch den Abgasstrom bereitgestellt. Gemäß einer weiteren Ausführungsform der Erfindung wird aus dem Abgasstrom nicht umgesetzter Sauerstoff entfernt. Gemäß einer Ausführungsform der Erfindung wird der Sauerstoff katalytisch abgetrennt oder durch Umsetzung mit einem Reduktionsmittel aus dem Abgasstrom entfernt. Gemäß einer Ausführungsform der Erfindung wird der durch den Rohwasserstoffstrom oder ersten Produktstrom bereitgestellte Wasserstoff als Reduktionsmittel verwendet.

Gemäß einer weiteren Ausführungsform der Erfindung ist vorgesehen, dass aus dem Rohwasserstoffstrom neben dem ersten Produktstrom ein erster Restgasstrom abgetrennt wird, der im Wesentlichen CO₂, und optional H₂, nicht umgesetztes CO und/oder nicht umgesetztes Methan umfasst, und der erste Restgasstrom in den ersten Teilstrom zurückgeführt wird. Gemäß einer weiteren Ausführungsform der Erfindung ist vorgesehen, dass der erste Restgasstrom durch Druck-Wechsel-Absorption unter Verwendung eines H₂-haltigen Spülgases abgetrennt wird, so dass der Restgasstrom zusätzlich H₂ umfasst. Vorteilhafterweise kann der im Restgasstrom enthaltene Wasserstoff in die Brennkammer des Reformers zurückgeführt werden, um dort durch Verbrennung die für den Reformierungsschritt erforderliche Wärme zu erzeugen.

Gemäß einem weiteren Aspekt der Erfindung wird eine Anlage zur Synthesegasherstellung zur Verfügung gestellt. Eine solche Anlage umfasst:
- ein Leitungssystem, das zum Führen eines Einsatzgasstroms ausgebildet ist,
- ein mit dem Leitungssystem in Strömungsverbindung stehenden Dampfreformer, der zumindest ein Reformerrohr und eine Brennkammer umfasst, wobei die Brennkammer dazu ausgebildet ist, einen Gasstrom umfassend einen Brennstoff in Gegenwart eines sauerstoffhaltigen Gasstroms unter Entstehung eines Abgasstroms zu verbrennen, und die dabei entstehende Hitze auf das zumindest eine Reformerrohr zu übertragen,
wobei das Leitungssystem weiterhin dazu ausbildet ist, den Einsatzgasstrom in einen Einsatzgashauptstrom und einen Einsatzgasteilstrom zu trennen, den Einsatzgashauptstrom in das zumindest eine Reformerrohr, den Einsatzgasteilstrom in die Brennkammer, und den Abgasstrom in den Einsatzgashauptstrom zu führen. Das zumindest eine Reformerrohr ist mit einem Katalysator ausgestattet, beispielsweise mit einem Katalysator umfassend Nickel oder Ruthenium.

Gemäß einer Ausführungsform der Erfindung ist vorgesehen, dass der Dampfreformer in Strömungsverbindung mit einem Fischer-Tropsch-Reaktor steht, der dazu ausgebildet ist, Synthesegas in Kohlenwasserstoffe umzuwandeln. Insbesondere umfasst ein solcher Reaktor zumindest einen Katalysator, der insbesondere auf einem Übergangsmetall wie etwa Cobalt, Eisen, Nickel oder Ruthenium basiert.

Gemäß einer weiteren Ausführungsform der Erfindung ist vorgesehen, dass der Fischer-Tropsch-Reaktor in Strömungsverbindung zu einer Trenneinheit steht, die dazu ausgebildet ist, einen Restgasstrom umfassend leichte C₁-C₄ Kohlenwasserstoffe und Synthesegas aus einem kohlenwasserstoffhaltigem Stoffstrom abzutrennen. Vorteilhafterweise ist das Leitungssystem dazu ausgebildet, den Restgasstrom in den Fischer-Tropsch-Reaktor und/oder in den Einsatzgashauptstrom zu führen.

Gemäß einer weiteren Ausführungsform der Erfindung ist vorgesehen, dass der Dampfreformer in Strömungsverbindung mit einem Wassergas-Shift-Reaktor steht, der dazu ausgebildet ist, CO und Wasser zu H₂ und CO₂ umzuwandeln.

Gemäß einer weiteren Ausführungsform der Erfindung steht der Wassergas-Shift-Reaktor in Strömungsverbindung mit einem Druck-Wechsel-Adsorber, der dazu ausgebildet ist, einen weiteren Restgasstrom umfassend CO₂ und optional H₂, CO und/oder Methan aus einem wasserstoffhaltigen Gasstrom abzutrennen. Vorteilhafterweise ist das Leitungssystem dazu ausgebildet, den weiteren Restgasstrom in den Einsatzgasteilstrom bzw. in die Brennkammer zu führen.

Gemäß einer weiteren Ausführungsform der Erfindung ist vorgesehen, dass die Trenneinheit in Strömungsverbindung mit einem Hydrierungsreaktor steht, der dazu ausgebildet ist, ungesättigte oder sauerstoffhaltige Kohlenwasserstoffverbindungen zu hydrieren.

Gemäß einer weiteren Ausführungsform steht der Hydrierungsreaktor in Strömungsverbindung mit dem Druck-Wechsel-Adsorber.

Gemäß einer weiteren Ausführungsform der Erfindung ist zwischen dem Dampfreformer und den Fischer-Tropsch-Reaktor und/oder zwischen dem Dampfreformer und dem Wassergas-Shift-Reaktor ein Wärmeübertrager angeordnet, der dazu ausgebildet ist, Wärme aus einem heißen Gasstrom auf Wasser unter Entstehung von Wasserdampf zu übertragen. Insbesondere umfasst der Wärmeübertrager zumindest ein Rohr, das von einem Mantel umschlossen wird, wobei das zu erhitzende Wasser bevorzugt rohrseitig, und der heiße Gasstrom mantelseitig geführt wird.

Gemäß einer weiteren Ausführungsform der Erfindung steht die Brennkammer in Strömungsverbindung mit einer Luftzerlegungseinheit, die dazu ausgebildet ist, Luft in Sauerstoff und Stickstoff zu zerlegen.

Gemäß einer alternativen Ausführungsform der Erfindung steht der Wassergas-Shift-Reaktor oder der Druck-Wechselabsorber in Strömungsverbindung mit einem Ammoniakreaktor, wobei der Ammoniakreaktor dazu ausgebildet ist, Wasserstoff und Stickstoff zu Ammoniak umzusetzen.

Gemäß einer weiteren Ausführungsform steht der Ammoniakreaktor in Strömungsverbindung mit der Luftzerlegungseinheit.

Gemäß einer weiteren Ausführungsform der Erfindung steht der Ammoniakreaktor in Strömungsverbindung mit einem Harnstoffreaktor, der dazu ausgebildet ist, Ammoniak mit Kohlendioxid zu Harnstoff umzusetzen.

Gemäß einer weiteren Ausführungsform der Erfindung steht die Brennkammer in Strömungsverbindung mit dem Harnstoffreaktor.

Gemäß einer Ausführungsform der Erfindung ist zwischen der Brennkammer und dem Harnstoffreaktor ein Sauerstoffabscheider angeordnet, der dazu ausgebildet ist, aus dem Abgas der Brennkammer Sauerstoff zu entfernen. Insbesondere ist der Sauerstoffabscheider dazu ausgebildet, Sauerstoff mit Wasserstoff zu Wasser verbrennen zu lassen.

Weitere Einzelheiten und Vorteile der Erfindung sollen durch die nachfolgenden Figurenbeschreibungen von Ausführungsbeispielen anhand der Figuren erläutert werden.

Es zeigen:
- Fig. 1: ein Schema einer Ausführungsform des erfindungsgemäßen Verfahrens;
- Fig. 2: ein Schema einer alternativen Ausführungsform des erfindungsgemäßen Verfahrens; und
- Fig. 3: ein Schema einer weiteren alternativen Ausführungsform des erfindungsgemäßen Verfahrens.

### Beispiele:

### Beispiel 1:

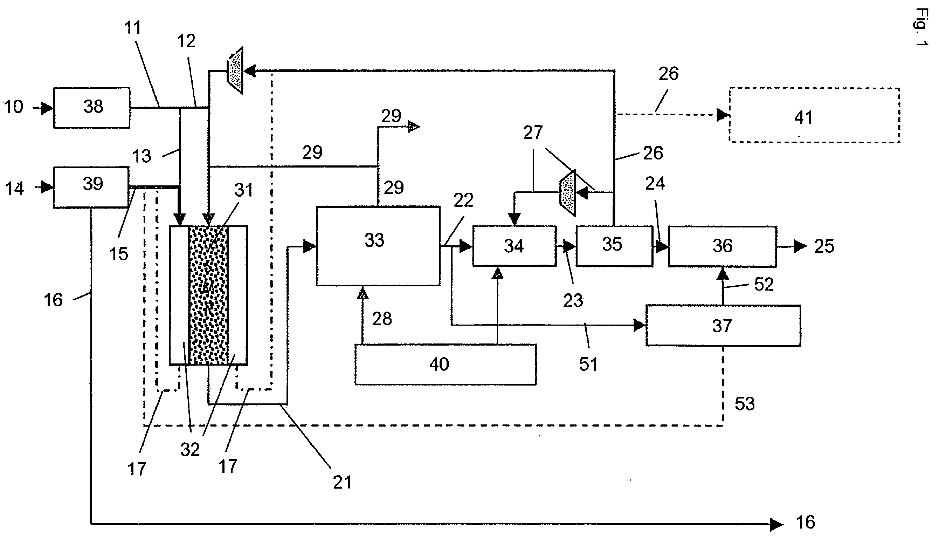

Figur 1 illustriert die Hauptschritte einer bevorzugten Verfahrensführung der Erfindung, eines Fischer-Tropsch (F-T)-Verfahrens, bei dem gasförmige Kohlenwasserstoffe in flüssige Kohlenwasserstoffe umgewandelt werden. Die Schritte bestehen im Wesentlichen aus einer Entschwefelung 38 des Einsatzgasstroms 10, einem Reformieren 31 des Einsatzgasstroms 11, 12, einem Kühlen des Reformats 21, und einer Fischer-Tropsch-Synthese 34 mit dem gekühlten Reformat 22.

Nach der Entschwefelung 38, bei der schwefelhaltige Verbindungen von einem kohlenwasserstoffhaltigen Einsatzgasstrom 10 wie beispielsweise Erdgas entfernt werden, wird der entschwefelte Einsatzgasstrom 11 in einen Einsatzgashauptstrom 12 (auch als erster Einsatzgasstrom bezeichnet) und einen Einsatzgasteilstrom 13 (auch als Teilstrom bezeichnet) getrennt. Der Einsatzgashauptstrom 12 wird in einen Dampfreformer 31 geführt, der eine Brennkammer 32 und mehrere Reformerrohre 31 umfasst. Die Reformerrohre 31 sind dabei mit einem oder mehreren geeigneten Katalysatoren ausgestattet, wie etwa einem Nickelkatalysator oder einem Rutheniumkatalysator. Der Einsatzgashauptstrom 12 strömt zusammen mit Wasserdampf 29 in die Reformerrohre 31 und wird dort zu einem Synthesegas 21 umfassend CO und H₂ umgesetzt, insbesondere bei einer Temperatur von 750°C bis 950°C und einem Druck von etwa 10 bar bis 40 bar, vorzugsweise bei einem Druck von 30 bar.

Das Synthesegas 21 aus den Reformerrohren 31 wird gekühlt 33, insbesondere zunächst auf eine Temperatur von 50°C bis 70°C, wobei Wasser 28 aus einem Reservoir 40 durch die entnommene Wärme unter Entstehung von gesättigtem Wasserdampf 29 erhitzt wird. Das gekühlte Synthesegas 22 wird dann in einem Abscheider getrocknet und anschließend wieder auf eine Temperatur von 150°C bis 220°C erhitzt. Das trockene Synthesegas 22 wird dann in einen Fischer-Tropsch-Reaktor 34 geleitet, in welchem CO und Wasserstoff zu einem Rohprodukt 23 umfassend Kohlenwasserstoffe unterschiedlicher Kettenlängen umgesetzt werden. Typischerweise enthält das Rohprodukt 23 leichte Kohlenwasserstoffe bzw. Flüssiggase, Rohbenzin, Diesel und Paraffine sowie nicht umgesetztes Synthesegas 22. Das nicht umgesetzte Synthesegas und die leichten Kohlenwasserstoffe werden vom Rohprodukt 23 der Fischer-Tropsch-Synthese 34 abgetrennt und über zwei verschiedenen Restgasströme 26, 27 (F-T-*tail gas*) recycelt: ein interner Restgasstrom 27 zum Fischer-Tropsch-Reaktor, und ein externer Restgasstrom 26 zurück in den Dampfreformer 31.

Bevorzugt wird ein Teil des Restgasstromes 26 abgetrennt. Dadurch können vorteilhafterweise Anhäufungen von inerten Verbindung im oben beschriebenen Recyclingkreislauf vermieden werden. Der abgetrennte Teil wird dabei vorteilhafter als Treibstoff benutzt (41) bzw. nutzbringend verbrannt.

Der Einsatzgasteilstrom 13 wird in die Brennkammer 32 des Dampfreformators geführt und mit Sauerstoff 15 als Oxidationsmittel verbrannt. Das resultierende Rauchgas bzw. Abgas 17 der Verbrennung, welches hauptsächlich CO₂ und Wasser und Spuren von H₂ enthält, wird zu einem Teil in den Reformer 31 bzw. den Einsatzgashauptstrom 12 zurückgeführt, insbesondere über den externen Restgasstrom 26 aus der Fischer-Tropsch-Synthese 34. Der andere Teil des Abgases 17 wird in die Brennkammer 32 bzw. den Einsatzgasteilstrom 13 zurückgeführt, um die Flammentemperatur zu regulieren.

Ein Teil 51 des gekühlten Synthesegases wird in einen Wassergas-Shift-Reaktor 37 geführt, in dem unter Zugabe von Wasserdampf CO zu CO₂ und Wasserstoff umgesetzt wird. Der Wasserstoff 52 wird anschließend über eine Druck-Wechsel-Absorption (pressure *swing absorption-*PSA) 37 als wasserstoffhaltiger Produktstrom 52 abgetrennt und zur Hydrierung 36 der Produkte 24 der Fischer-Tropsch-Synthese 34 verwendet. Das aus der Druck-Wechselabsorption resultierende Restgas (*tail gas*) 53 wird als Brennstoff in der Brennkammer 32 des Dampfreformators verwendet. Vorteilhafterweise wird die Druck-Wechsel-Absorption 37 mit einem wasserstoffhaltigen Spülgas durchgeführt, das vorteilhafterweise zumindest zum Teil durch den wasserstoffhaltigen Produktstrom 52 bereitgestellt wird. Durch die Verwendung des wasserstoffhaltigen Spülgases, enthält das Restgas 53 neben CO₂ auch beachtliche Mengen an Wasserstoff, der wie oben beschrieben, als Brennstoff für den Dampfreformator verwendet werden kann.

Durch die vorgeschlagene Verfahrensführung erhöht sich die Kohlenstoffeffizienz des Verfahrens. Des Weiteren erlaubt die vorgeschlagene Verfahrensführung eine effektivere Nutzung der entstehenden Wärme.

Weitere Vorteile sind:
- Die Rückführung des Restgases aus der Fischer-Tropsch-Synthese (FT-*tail* gas) ohne weitere Aufarbeitung und/oder des Abgases zum Synthesegasschritt reduziert den Partialdruck von CO, welches verantwortlich für die beta-Deaktivierung des Nickelkatalysators ist;
- Die Rückführung des Abgases erhöht die Ausbeute des Synthesegasschrittes, d.h. mehr H₂ und CO Moleküle entstehen pro Volumen des eingesetzten Erdgases;
- Kein Energieexport;
- Geringere CO₂-Emission.

Die folgende Tabelle fasst ein paar der Schlüsselparameter zusammen, die durch ein konventionelles SMR-Verfahren (Standard) und durch die vorgeschlagene Verfahrensführung (oxy-comb) erzielt wurden. Der Vergleich wurde auf Basis des gleichen Erdgases gemacht.

| Verfahren | Standard | Oxy-Comb |
|---|---|---|
| Erdgaseinsatz (Ncum/h) | 43500 | 43500 |
| Brennstoffeinsatz (Ncum/h) | 4000 | 800 |
| Gesamt (Ncum/h) | 47500 | 44300 |
| Sauerstoff (Ncum/h) | 0 | 42000 |
| Restgasbrennstoff (tail gas fuel) | 29000 | 16900 |
| SMR Feuernutzleistung (MW) | 117 | 134,3 |
| Produkt | | |
| H2+ CO (Ncum/h) | 71383 | 121913 |
| Nettoenergie (MW) | 15,1 | -11,2 |
| Erdgasäquivalente | 1991,0 | -1477,0 |
| Stickstoffprodukt (Ncum/h) | 0,00 | 4848 |
| CO2 Emission (MTPD) | 45510 | 45776 |
| | 967,0 | 663,0 |
| Verhältnis erzeugtes Synthesegas/ eingesetztes Erdgas | 2,0 | 2,7 |

### Beispiel 2:

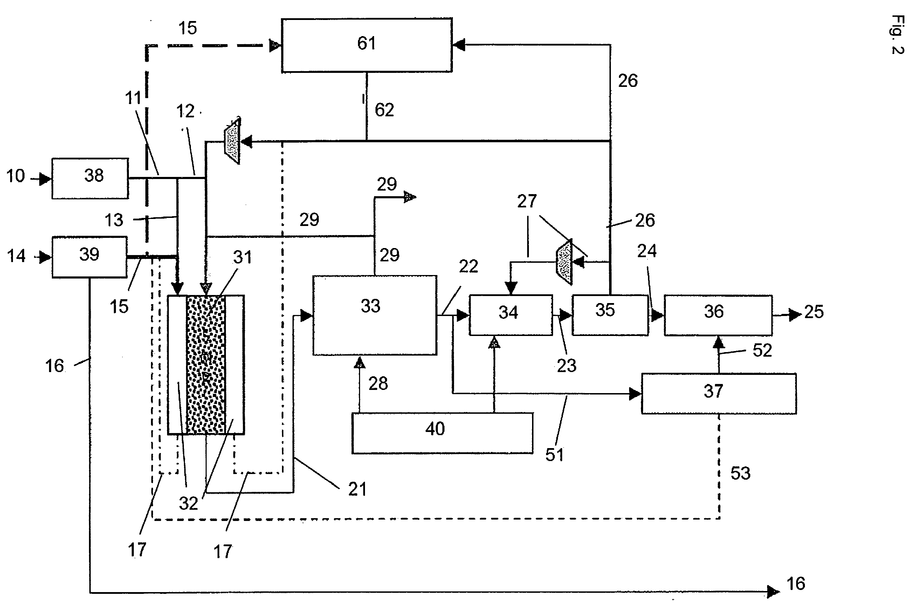

Figur 2 illustriert eine Weiterentwicklung der erfindungsgemäßen Verfahrensführung aus Beispiel 1. Demnach ist vorgesehen, dass ein Teil des Restgases 26, welches aus dem Rohprodukt 23 der Fischer-Tropsch-Synthese 34 abgetrennt wird, zur Energieerzeugung verbrannt 61 wird, wobei durch die Verbrennung 61 Wasserdampf 29 zur Energieerzeugung bereitgestellt wird. Die bei dieser Verbrennung 61 entstehenden Rauch- bzw. Abgase 62 werden nach Wasserabscheidung in den Reformer 31 bzw. in den Einsatzgashauptstrom 12 oder den Restgasstrom 26 aus der Fischer-Tropsch-Synthese 34 zurückgeführt. Alternativ kann für diesen Zweck auch ein Teil des Einsatzgasteilstromes 13 verwendet werden. Vorteilhafterweise kann das Verfahren durch eine solche Führung energieneutral betrieben werden.

### Beispiel 3:

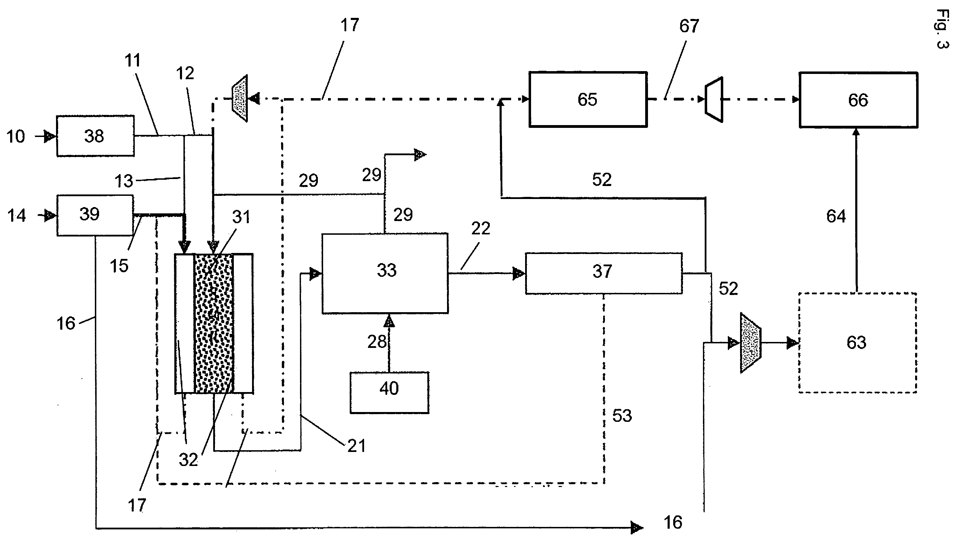

Das erfindungsgemäße Verfahren kann vorteilhafterweise auch in anderen Verfahren, als der Fischer-Tropsch-Synthese 34, verwendet werden. Figur 3 zeigt eine alternative Ausführungsform der Erfindung, wobei das erzeugte Synthesegas 22 ausschließlich in eine Wassergas-Shift-Reaktion 37 eingesetzt wird. Das resultierende wasserstoffreiche Produktgas 52 wird dabei anschließend in der Synthese 63 von Ammoniak 64 verwendet, in welcher elementarer Wasserstoff 52 und Stickstoff 16 zu Ammoniak 64 umgesetzt wird. Vorteilhafterweise wird der dafür notwendige elementare Stickstoff 16 durch die oben beschriebene Luftzerlegung 39 bereitgestellt.

Das synthetisierte Ammoniak 64 kann weiter zu Harnstoff umgesetzt werden, wobei das dafür benötigte Kohlendioxid vorteilhafterweise durch einen der oben beschriebenen Abgasströme 17 bereitgestellt wird. Der Abgasstrom 17 enthält neben CO₂ auch noch bei der Verbrennung 32 nicht umgesetzten Sauerstoff in einer Konzentration von typischerweise 1 Vol. % bis 3 Vol. %. Der Sauerstoff wird vor der Verwendung des Abstroms 17 in der Harnstoffsynthese 66 abgetrennt 65, um unvorteilhafte Reaktionen des Sauerstoffs während der Harnstoffsynthese zu vermeiden. Dabei wird der Sauerstoff entweder katalytisch abgetrennt oder mit einem Reduktionsmittel wie Wasserstoff 52 umgesetzt, wobei das im letzteren Falle entstehende Wasser anschließend abgetrennt wird. Der nun im Wesentlichen sauerstofffreie, CO₂-haltige Gasstrom 67 wird anschließend komprimiert und mit dem synthetisierten Ammoniak 64 zu Harnstoff umgesetzt 66.

**Bezugszeichenliste**

| | |
|---|---|
| 10 | Erdgas |
| 11 | Erdgas entschwefelt bzw. erster Einsatzgasstrom |
| 12 | Einsatzgashauptstrom bzw. erster Einsatzgasstrom |
| 13 | Einsatzgasteilstrom bzw. Teilstrom |
| 14 | Luft |
| 15 | Gasstrom mit 95 Vol. % bis 99 Vol. % Sauerstoff |
| 16 | Stickstoff |
| 17 | Rauchgas/Abgas |
| 21 | Heißes Rohsynthesegas |
| 22 | Gekühltes Rohsynthesegas |
| 23 | F-T (Fischer-Tropsch)-Rohprodukt |
| 24 | F-T-Rohprodukt ohne Synthesegas und leichte Kohlenwasserstoffe |
| 25 | Hydriertes F-T-Produkt |
| 26 | Restgas/F-T *tailgas* (Synthesegas und leichte Kohlenwasserstoffe) |
| 27 | Recyclingstrom in F-T-Synthese |
| 28 | Wasser |
| 29 | Wasserdampf |
| 31 | Reformerrohre |
| 32 | Brennkammer |
| 33 | Wärmeübertrager |
| 34 | Fischer-Tropsch-Reaktor |
| 35 | Trenneinheit |
| 36 | Hydrierungsreaktor |
| 37 | Wassergas-Shift-Reaktor/PSA |
| 38 | Entschwefelungseinheit |
| 39 | Luftzerlegungseinheit |
| 40 | Wasserreservoir |
| 41 | Brennstoffsystem |
| 51 | Gekühlter Synthesegasstrom |
| 52 | Wasserstoff |
| 53 | Restgas (*tail gas*) der PSA |
| 61 | Hilfsboiler |
| 62 | Abgas aus dem Hilfsboiler |
| 63 | Ammoniaksynthese |
| 64 | Ammoniak |
| 65 | Sauerstoffabtrennung |
| 66 | Harnstoffsynthese |
| 67 | Im Wesentlichen sauerstofffreies Abgas |

## Patentansprüche

1. Verfahren zur Erzeugung von Synthesegas (21, 22), umfassend die Schritte:
- Bereitstellen eines ersten Einsatzgasstromes (11, 12) umfassend Methan,
- Umsetzen des ersten Einsatzgasstromes (11, 12) mit Wasserdampf (29) in einem Reformierungsschritt (31) unter Erhalt eines Synthesegasstroms (21, 22) umfassend CO und H₂,
**dadurch gekennzeichnet, dass**
- aus dem ersten Einsatzgasstrom (11) vor dem Reformierungsschritt (31) mindestens ein erster Teilstrom (13) abgetrennt wird,
- der erste Teilstrom (13) anschließend mit einem zweiten Einsatzgasstrom (15) umfassend mindestens 95 Vol. % Sauerstoff zu einem Abgasstrom (17) umfassend CO₂ und Wasser verbrannt wird, und
- der Abgasstrom (17) zumindest zu einem Teil in den ersten Einsatzgasstrom (12) stromauf des Reformierungsschritts (31) zurückgeführt wird.

2. Verfahren nach Anspruch 1, **dadurch gekennzeichnet, dass** die bei der Verbrennung (32) des ersten Teilstroms (13) entstehende Wärme auf den Reformierungsschritt (31) übertragen wird.

3. Verfahren nach Anspruch 1 oder 2, **dadurch gekennzeichnet, dass** der Abgasstrom (17) zu einem anderen Teil in den ersten Teilstrom (13) zurückgeführt wird.

4. Verfahren nach einem der Ansprüche 1 bis 3, **dadurch gekennzeichnet, dass** der zweite Einsatzgasstrom (15) durch die Gastrennung (39) von Luft (14) bereitgestellt wird, wobei durch die Gastrennung (39) zusätzlich ein im Wesentlichen Stickstoff umfassender dritter Einsatzgasstrom (16) bereitgestellt wird.

5. Verfahren nach einem der Ansprüche 1 bis 4, **dadurch gekennzeichnet, dass** der Synthesegasstrom (21) umfassend CO und H₂ unter Erzeugung von Wasserdampf (29) gekühlt wird.

6. Verfahren nach einem der Ansprüche 1 bis 5, **dadurch gekennzeichnet, dass** aus dem Synthesegasstrom (21, 22) umfassend CO und H₂ zumindest ein zweiter Teilstrom (51) abgetrennt wird, der in einem Wassergas-Shift-Reaktionsschritt (37) zu einem Rohwasserstoffstrom umgesetzt wird, wobei CO und Wasser zu CO₂ und H₂ umgesetzt werden.

7. Verfahren nach Anspruch 6, **dadurch gekennzeichnet, dass** aus dem Rohwasserstoffstrom unter Erhalt eines ersten Produktstroms (52), der im Wesentlichen H₂ umfasst, ein erster Restgasstrom (53), der CO₂, und H₂, nicht umgesetztes CO und/oder nicht umgesetztes Methan aufweist, abgetrennt wird, und der erste Restgasstrom (53) in den ersten Teilstrom (13) zurückgeführt wird, wobei der erste Restgasstrom (53) durch Druck-Wechsel-Absorption (37) unter Verwendung eines H₂-haltigen Spülgases abgetrennt wird, so dass der Restgasstrom (53) zusätzlich H₂ umfasst.

8. Verfahren nach einem der Ansprüche 1 bis 7, **dadurch gekennzeichnet, dass** der Synthesegasstrom (21, 22) umfassend CO und H₂ in einem Fischer-Tropsch-Syntheseschritt (34) zu einem Rohproduktstrom (23) umgesetzt wird, der eine Mischung aus mindestens einem leichten C₁-C₄ Kohlenwasserstoff, mindestens einem schweren Kohlenwasserstoff mit mehr als 4 Kohlenstoffatomen und nicht umgesetzten Synthesegas umfassend CO und H₂ aufweist.

9. Verfahren nach Anspruch 8, **dadurch gekennzeichnet, dass** aus dem Rohproduktstrom (23) ein zweiter Restgasstrom (26) umfassend den mindestens einen leichten C₁-C₄ Kohlenwasserstoff und das nicht umgesetzte Synthesegas abgetrennt wird, und der zweite Restgasstrom (23) in den ersten Einsatzgasstrom (11, 12) zurückgeführt wird.

10. Verfahren nach Anspruch 9, **dadurch gekennzeichnet, dass** aus dem zweiten Restgasstrom (26) ein zweiter Teilstrom (27) abgetrennt wird, und der zweite Teilstrom (27) in den Fischer-Tropsch-Syntheseschritt (34) zurückgeführt wird.

11. Verfahren nach Anspruch 7 und einem der Ansprüche 8 bis 10, **dadurch gekennzeichnet, dass** der erste Produktstrom (52) umfassend im Wesentlichen H₂ zumindest zum Teil in den Rohproduktstrom (24) nach Abtrennen des zweiten Restgasstromes (26) geführt wird.

12. Verfahren nach einem der Ansprüche 9 bis 11, **dadurch gekennzeichnet, dass** aus dem zweiten Restgasstrom (26) ein dritter Teilstrom abgetrennt wird, der unter Erzeugung von Wasserdampf verbrannt (61) wird, wobei das resultierende Abgas (62) der Verbrennung (61) zumindest zum Teil in den Reformierungsschritt (31) über den zweiten Restgasstrom (26) zurückgeführt wird.

13. Verfahren zur Herstellung von Ammoniak, umfassend die Schritte
- Bereitstellen eines ersten Einsatzgasstromes (11, 12) umfassend Methan,
- Umsetzen des ersten Einsatzgasstromes (11, 12) mit Wasserdampf (29) in einem Reformierungsschritt (31) unter Erhalt eines Synthesegasstroms (21, 22) umfassend CO und H₂, wobei
• aus dem ersten Einsatzgasstrom (11) vor dem Reformierungsschritt (31) mindestens ein erster Teilstrom (13) abgetrennt wird,
• der erste Teilstrom (13) anschließend mit einem zweiten Einsatzgasstrom (15) umfassend mindestens 95 Vol. % Sauerstoff zu einem Abgasstrom (17) umfassend CO₂ und Wasser verbrannt wird,
• der Abgasstrom (17) zumindest zu einem Teil in den ersten Einsatzgasstrom (12) stromauf des Reformierungsschritts (31) zurückgeführt wird, und
• optional der zweite Einsatzgasstrom (15) durch die Gastrennung (39) von Luft (14) bereitgestellt wird, wobei durch die Gastrennung (39) zusätzlich ein dritter Einsatzgasstrom (16) umfassend im Wesentlichen Stickstoff bereitgestellt wird,
- Umsetzen von zumindest einem Teil des Synthesegasstroms umfassend CO und H₂ in einer Wassergas-Shift-Reaktion (37) zu einem Rohwasserstoffstrom, wobei CO und Wasser zu CO₂ und H₂ umgesetzt werden, und optional Abtrennen eines ersten Produktstromes (52) umfassend im Wesentlichen H₂ aus dem Rohwasserstoffstrom,
- Umsetzen von Wasserstoff (52) und Stickstoff (16) zu Ammoniak (64), wobei der Wasserstoff (52) durch den Rohwasserstoffstrom oder ersten Produktstrom (52) bereitgestellt wird, und der Stickstoff (16) durch den dritten Einsatzgasstrom (16) bereitgestellt wird.

14. Verfahren zur Herstellung von Harnstoff, umfassend die Schritte:
- Bereitstellen eines ersten Einsatzgasstromes (11, 12) umfassend Methan,
- Umsetzen des ersten Einsatzgasstromes (11, 12) mit Wasserdampf (29) in einem Reformierungsschritt (31) unter Erhalt eines Synthesegasstroms (21, 22) umfassend CO und H₂, wobei
• aus dem ersten Einsatzgasstrom (11) vor dem Reformierungsschritt (31) mindestens ein erster Teilstrom (13) abgetrennt wird,
• der erste Teilstrom (13) anschließend mit einem zweiten Einsatzgasstrom (15) umfassend mindestens 95 Vol. % Sauerstoff zu einem Abgasstrom (17) umfassend CO₂ und Wasser verbrannt wird,
• der Abgasstrom (17) zumindest zu einem Teil in den ersten Einsatzgasstrom (12) stromauf des Reformierungsschritts (31) zurückgeführt wird, und
• optional der zweite Einsatzgasstrom (15) durch die Gastrennung (39) von Luft (14) bereitgestellt wird, wobei durch die Gastrennung (39) zusätzlich ein dritter Einsatzgasstrom (16) umfassend im Wesentlichen Stickstoff bereitgestellt wird
- Umsetzen von zumindest einem Teil des Synthesegasstroms umfassend CO und H₂ in einer Wassergas-Shift-Reaktion (37) zu einem Rohwasserstoffstrom, wobei CO und Wasser zu CO₂ und H₂ umgesetzt werden, und optional Abtrennen eines ersten Produktstromes (52) umfassend im Wesentlichen H₂ aus dem Rohwasserstoffstrom,
- Umsetzen von Wasserstoff (52) und Stickstoff (16) zu Ammoniak (64), wobei der Wasserstoff (52) durch den Rohwasserstoffstrom oder ersten Produktstrom bereitgestellt wird, und der Stickstoff (16) durch den dritten Einsatzgasstrom (16) bereitgestellt wird, und
- Umsetzen des Ammoniaks (64) und CO₂ (17) zu Harnstoff, wobei CO₂ durch den Abgasstrom (17) bereitgestellt wird, wobei sich im Abgasstrom befindlicher, nicht umgesetzter Sauerstoff durch Wasserstoff (52) zu Wasser reduziert wird, und wobei der Wasserstoff (52) für die Reduktion des Sauerstoffs durch den Rohwasserstoffstrom oder ersten Produktstrom (52) bereitgestellt wird.

15. Anlage zur Synthesegasherstellung umfassend,
- ein Leitungssystem, das zum Führen eines Einsatzgasstroms (11,12,15, 16) ausgebildet ist,
- einen mit dem Leitungssystem in Strömungsverbindung stehenden Dampfreformer, der zumindest ein Reformerrohr (31) und eine Brennkammer (32) umfasst, wobei die Brennkammer (32) dazu ausgebildet ist, einen Gasstrom (11,12) umfassend einen Brennstoff in Gegenwart eines sauerstoffhaltigen Gasstroms (15) unter Entstehung eines Abgasstroms (17) zu verbrennen, und die dabei entstehende Hitze auf das zumindest eine Reformerrohr (31) zu übertragen,
**dadurch gekennzeichnet, dass**
das Leitungssystem weiterhin dazu ausbildet ist, den Einsatzgasstrom (11) in einen Einsatzgashauptstrom (12) und einen Einsatzgasteilstrom (13) zu trennen, den Einsatzgashauptstrom (12) in das zumindest eine Reformerrohr (31), den Einsatzgasteilstrom (13) in die Brennkammer (32) zu führen, und den Abgasstrom (17) in den Einsatzgashauptstrom (12) zu führen.
